(19) Europäisches Patentamt
European Patent Office
Office européen des brevets

(11) Veröffentlichungsnummer: **0 418 198 A1**

(12) **EUROPÄISCHE PATENTANMELDUNG**

(21) Anmeldenummer: **90810667.7**

(51) Int. Cl.5: **C07D 249/20**, D06M 13/352

(22) Anmeldetag: **04.09.90**

(30) Priorität: **11.09.89 CH 3290/89**

(43) Veröffentlichungstag der Anmeldung:
**20.03.91 Patentblatt 91/12**

(84) Benannte Vertragsstaaten:
**CH DE FR GB IT LI**

(71) Anmelder: **CIBA-GEIGY AG**
**Klybeckstrasse 141**
**CH-4002 Basel(CH)**

(72) Erfinder: **Burdeska, Kurt, Dr.**
**Laufenburgerstrasse 30**
**CH-4058 Basel(CH)**
Erfinder: **Reinert, Gerhard, Dr.**
**Weiherweg 1/7**
**CH-4123 Allschwil(CH)**

(54) **Monosulfonierte 2-(2'-Hydroxyphenyl)benzotriazole.**

(57) Monosulfonierte 2-(2'-Hydroxyphenyl)-benzotriazole der Formel

(1) 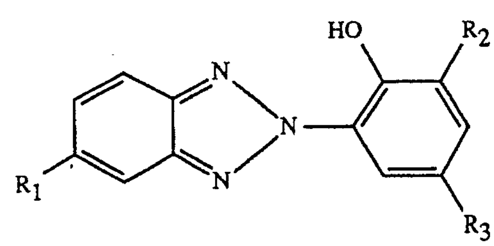

worin $R_1$ Wasserstoff oder Chlor und von $R_2$ und $R_3$ der eine Chlor oder Niederalkyl und der andere einen Rest der Formel

(2) 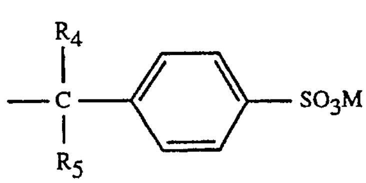 bedeutet, worin

$R_4$ und $R_5$ unabhängig voneinander Wasserstoff oder Methyl und M Wasserstoff, Alkalimetall oder Ammonium bedeuten.

Die Verbindungen der Formel (1) finden Verwendung als UV-Absorber für Polyamidfasern, Wolle und deren Mischungen.

EP 0 418 198 A1

## MONOSULFONIERTE 2-(2′-HYDROXYPHENYL)BENZOTRIAZOLE

Die vorliegende Erfindung betrifft monosulfonierte 2-(2′-Hydroxyphenyl)benzotriazole, Verfahren zu ihrer Herstellung und ihre Verwendung als UV-Absorber für Polyamidfasern, Wolle und deren Mischungen.

Die erfindungsgemässen monosulfonierten 2-(2′-Hydroxyphenyl)benzotriazole entsprechen der allgemeinen Formel

(1) 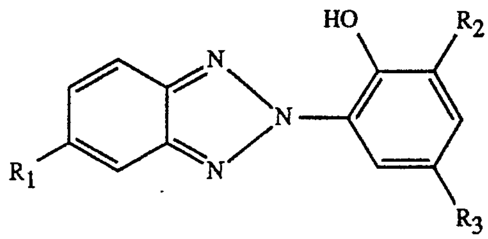 ,

worin $R_1$ Wasserstoff oder Chlor und von $R_2$ und $R_3$ der eine Chlor oder Niederalkyl und der andere einen Rest der Formel

(2) 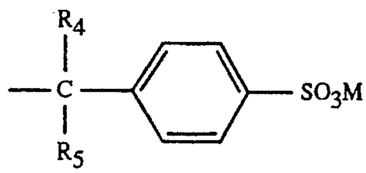

bedeutet, worin $R_4$ und $R_5$ unabhängig voneinander Wasserstoff oder Methyl und M Wasserstoff, Alkalimetall oder Ammonium bedeuten.

Niederalkyl stellen bei der Definition von $R_2$ und $R_3$ solche Gruppen dar, die 1 bis 5, insbesondere 1 bis 4 Kohlenstoffatome aufweisen. Beispiele für derartige Gruppen sind Methyl, Ethyl, n-Propyl, Isopropyl, n-Butyl, sek.-Butyl, tert.Butyl, Amyl, Isoamyl oder tert.Amyl.

M bedeutet insbesondere Kalium oder Natrium.

Von besonderem Interesse sind Verbindungen, bei denen $R_2$ Niederalkyl und $R_3$ einen Rest der Formel (2) bedeuten, bzw. Verbindungen, bei denen $R_2$ einen Rest der Formel (2) und $R_3$ Niederalkyl bedeuten.

Die Verbindungen der Formel (1) werden hergestellt, indem man eine Verbindung der Formel (3)

(3) 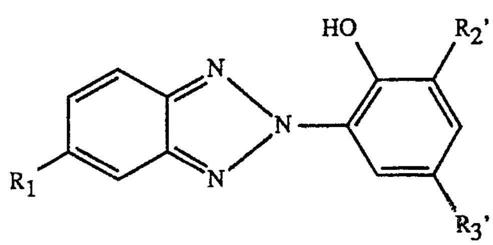 ,

worin $R_1$ die in Formel (1) angegebene Bedeutung hat und von $R_2'$ und $R_3'$ der eine Chlor oder Niederalkyl und der andere einen Rest der Formel (4)

(4) 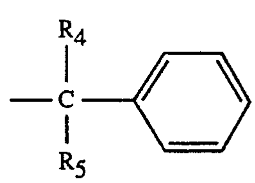

bedeutet, worin $R_4$ und $R_5$ die in Formel (2) angegebene Bedeutung haben, mit Schwefelsäure sulfoniert.

Die für die Sulfonierung benötigte Schwefelsäure kann als konzentrierte Schwefelsäure, Schwefelsäuremonohydrat oder Oleum von unterschiedlichem $SO_3$-Gehalt zur Anwendung kommen.

Die Reaktionstemperaturen können zwischen 0 und 30 °C schwanken. Verwendet man Schwefelsäuremonhydrat, so werden Temperaturen von 0 bis 30 °C, insbesondere 20 bis 25 °C bevorzugt. Verwendet man dagegen 5%-iges Oleum, so sind Temperaturen von 0 bis 30 °C, insbesondere 15 bis 25 °C angezeigt.

Mit dem erfindungsgemässen Verfahren lassen sich einheitliche monosulfonierte 2-(2'-Hydroxyphenyl)-benzotriazole entsprechend Formel (1) in einfacher Weise herstellen.

Monosulfonierte 2-(2'-Hydroxyphenyl)benzotriazole der Formel (1) finden Verwendung als UV-Absorber für Polyamidfasern, Wolle und deren Mischungen. Sie können weiter auch zur Verhinderung der Vergilbung von Substraten, wie beispielsweise Polyamidfasem, die mit Fleckenschutzmitteln, wie z.B Syntanen, ausgerüstet sind, eingesetzt werden.

Die erfindungsgemässen UV-Absorber können auch zusammen mit anderen Stabilisatoren eingesetzt werden.

Die folgenden Beispiele dienen zur Erläuterung der Erfindung. Die Temperaturen sind in °C angegeben.

Herstellungsbeispiele:

Beispiel 1: 12,7 g 2-(2'-Hydroxy-3'-methyl-5'-α-α-dimethylbenzylphenyl)-benzotriazol (Schmelzpunkt: 155 - 156°) werden bei Raumtemperatur in 20 Minuten in 120 g 5%-igem Oleum eingetragen und noch weitere 40 Minuten bei 20-25° gerührt. Anschliessend wird die gelbe Lösung in 350 ml 10%-iger NaCl-Lösung eingerührt, wobei die Temperatur auf ca. 75° ansteigt. Die ausgefallene Säure wird bei Raumtemperatur abfiltriert, danach in 200 ml Wasser angeschlämmt und mit 30%-iger Natronlauge auf den pH-Wert 7 eingestellt. Das Produkt wird abfiltriert, mit 10%-iger NaCl-Lösung, dann mit einem Aceton/Wasser-Gemisch im Verhältnis 7:3 gewaschen und bei 80° im Vakuum getrocknet. Man erhält 13,8 g eines farblosen Produktes der Formel

(101)

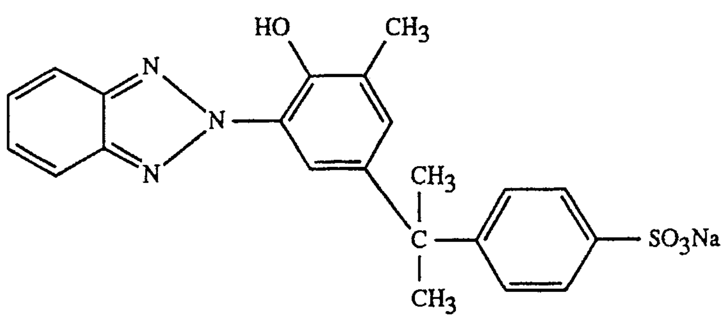

Verwendet man anstelle der 12,7 g 2-(2'-Hydroxy-3'-methyl-5'-α-α-dimethylbenzylphenyl)-benzotriazol 15,5 g 2-(2'-Hydroxy-3'-α-α-dimethylbenzylphenyl-5'-methylphenyl)-benzotriazol(Schmelzpunkt: 171-173°), erhält man, bei sonst gleicher Arbeitsweise 16,5 g des Produktes der Formel

(102)

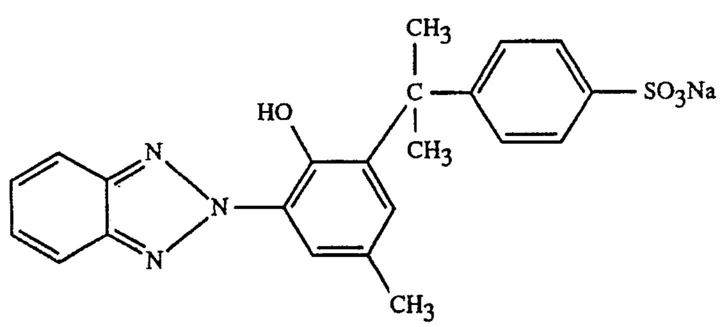

Beispiel 2: 18 g 2-(2'-Hydroxy-3'α-phenylethyl-5'-methylphenyl)-benzotriazol (Schmelzpunkt 120°) werden unter gutem Rühren innerhalb von 10 Minuten in 180 g Schwefelsäuremonohydrat eingetragen. Die Temperatur soll dabei 25° nicht überschreiten. Anschliessend wird die gelbe Lösung eine Stunde bei 23-26° gerührt. Danach wird die Lösung in ca. 5 Minuten in 400 ml einer 5%-igen NaCl-Lösung eingerührt, wobei die Temperatur auf ca. 75° ansteigt. Nach Zugabe von 10 g NaCl lässt man die Mischung auf Raumtemperatur abkühlen, filtriert das ausgefallene Produkt und trägt es in 400 ml Wasser ein. Die Säure wird mit 30%-iger Natronlauge auf den pH-Wert 7 eingestellt. Anschliessend wird auf 80° erhitzt, wobei eine Lösung entsteht. Nach Zugabe von 20 g NaCl lässt man auf Raumtemperatur abkühlen, filtriert das ausgefallene Produkt ab, wäscht zuerst mit 2%-iger NaCl-Lösung, dann mit einem Methanol/Wasser-Gemisch im Verhältnis 7:3 und trocknet anschliessend bei 80° im Vakuum.

Man erhält 21,5 g des Produktes der Formel

(103)

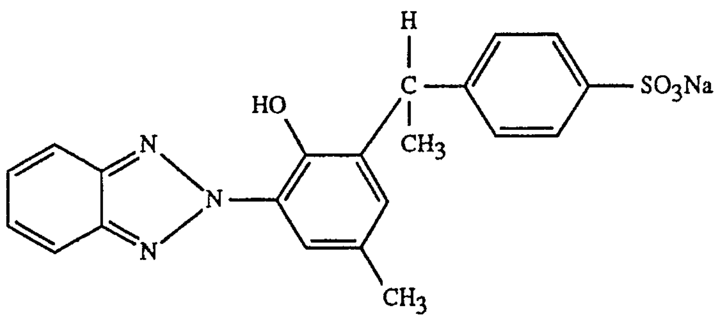

Applikationsbeispiele

Beispiel 3: Vier Muster eines Woll-Sèrge-Gewebes von je 10 g werden in einem offenen Färbeapparat, z.B. einem ®Ahiba, bei einem Flottenverhältnis von 1:30 in 2 Flotten "blind gefärbt" (ohne Farbstoff) und in 2 Flotten gefärbt.

Zunächst bereitet man 4 gleiche Flotten mit folgenden Zusätzen:

1,5 g/l kristallines Natriumacetat
2,0 % 80%-ige Essigsäure
5,0 % kalziniertes Glaubersalz
1,0 % eines Egalisiermittels

In diesen auf 40° erwärmten Flotten behandelt man die Wollmuster 10 Minuten, wobei sich ein pH-Wert von 4,5 einstellt.

Man entnimmt die Muster den Flotten und fügt folgende Zusätze hinzu:

Flotte 1: (Blindfärbung 1): kein Zusatz
Flotte 2: (Blindfärbung 2): 1 % der Verbindung der Formel (103)
Flotte 3: (Färbung 1): Folgende Farbstoffe in gelöster Form:
0,005 % der Verbindung der Formel

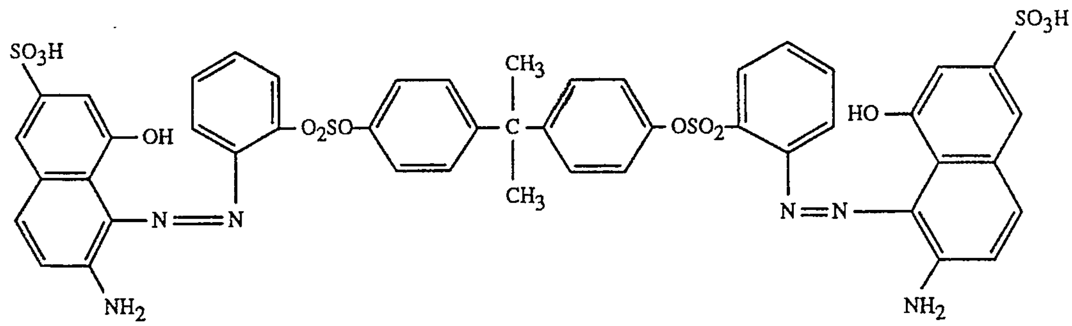

(104)

0,01 % der Verbindung der Formel

(105) 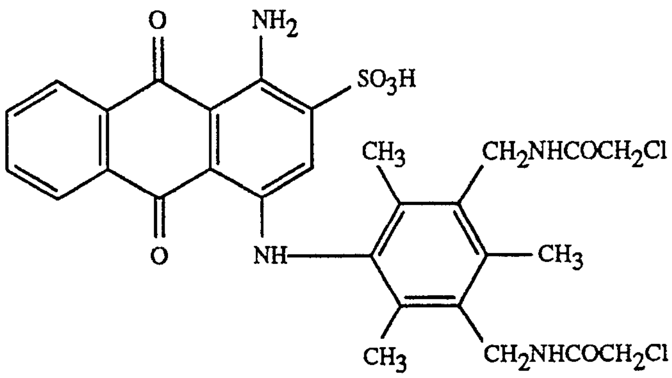

Flotte 4 (Färbung 2): Diese Flotte enthält 1 % der Verbindung der Formel (103) sowie 0,005 % des Farbstoffes der Formel (104) und 0,01 % des Farbstoffes der Formel (105).

Man gibt die Muster in die Färbeflotte zurück, behandelt zunächst weitere 10 Minuten bei 40° und erhitzt sodann mit 1,5°/Minute auf 90°. Man behandelt bei dieser Temperatur 45 Minuten, kühlt auf 60° ab, spült mit kaltem Wasser, zentrifugiert und trocknet bei 70°. Aus der Tabelle ist ersichtlich, dass die Mitverwendung von Verbindung (103) zu einer geringeren Vergilbung der Wolle führt und höhere Lichtechtheiten bei Färbungen bewirkt. Die beiden Blindfärbungen sowie das unbehandelte Ausgangsmaterial werden in einer Belichtungsapparatur mit Xenonlampe nach DIN 75.202 72 Stunden und 144 Stunden (= 1 bzw. 2 Testcyclen) belichtet. Danach wird der Gelbwert nach DIN 6167 aus den farbmetrischen Daten ermittelt.

Von den beiden Färbungen werden die Lichtechtheiten nach SN-ISO 105-B02 (= Xenon), sowie nach DIN 75.202 (= Fakra) bestimmt. Die Ergebnisse sind in Tabelle 1 aufgeführt.

Tabelle 1:

| Behandlung | Gelbwerte | Lichtechtheiten | |
|---|---|---|---|
| | 0; 72 h;144 h | XENON | FAKRA |
| Blindfärbung 1 | 15,9/23,3/29,2 | — | — |
| Blindfärbung 2 | 17,0/19,9/24,3 | — | — |
| Färbung 1 | — | 5 | 2 GH |
| Färbung 2 | — | 6 | 3-4 GH |
| Ausgangsmaterial | 15,3/21,7/26,8 | — | — |
| Bewertung: XENON gegen Blaumassstab<br>FAKRA gegen Graumassstab | | | |

Beispiel 4: 4 Muster eines Polyamid-6-Webtrikots werden in einem offenen Färbeapparat, z.B. einem ®AHIBA, bei einem Flottenverhältnis von 1:30 in 4 Flotten behandelt. Alle Flotten enthalten 1 g/l Ammoniumsulfat und folgende Farbstoffe (die Prozenangaben der Farbstoffe beziehen sich auf das Gewicht des Fasermaterials): 0,04 % des Farbstoffes der Formel

(106) 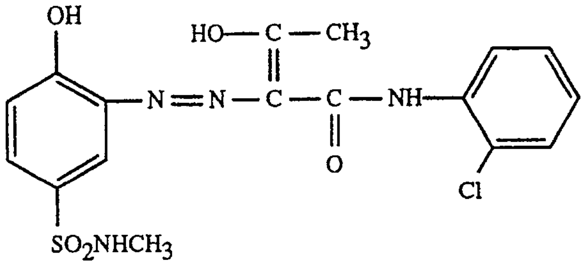 1:2-Co-Komplex (gelb)

0,025 % des Farbstoffes der Formel

(107)

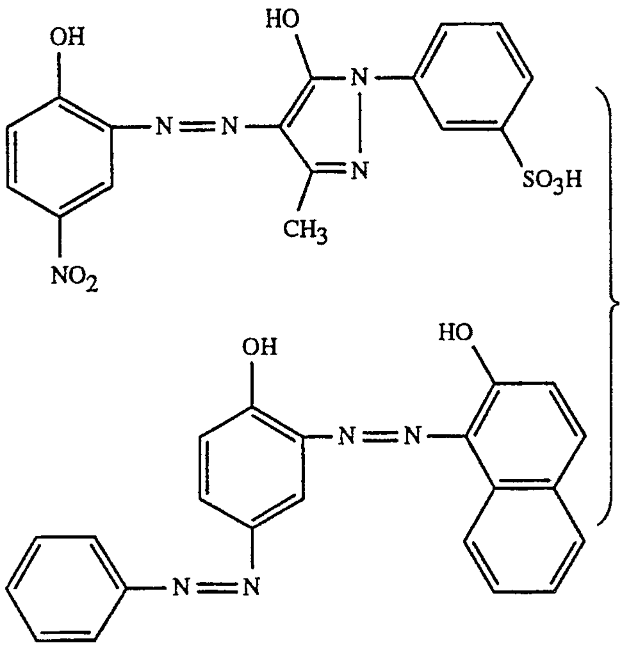

1:2-Cr-Komplex (braun)

0,003 % des Farbstoffes der Formel

(108)

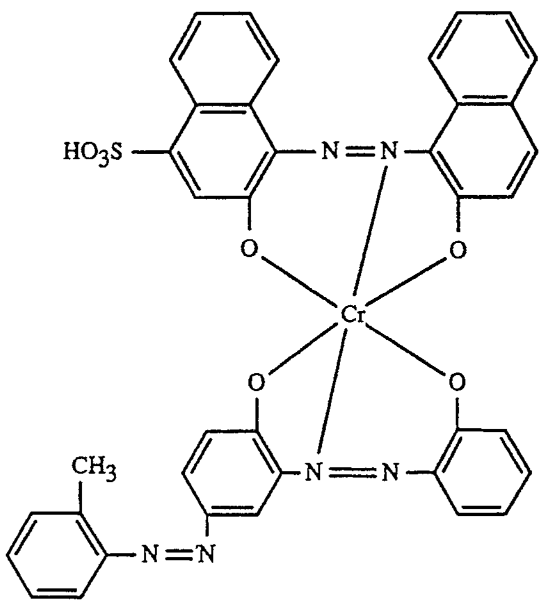

1:2-Cr-Komplex (schwarz)

Den einzelnen Flotten werden folgende Zusätze zugeben:

Flotte 1: keine weiteren Zusätze

Flotte 2: 0,5 % der Verbindung der Formel (103)

Flotte 3: 0,03 % der Verbindung der Formel

(109) 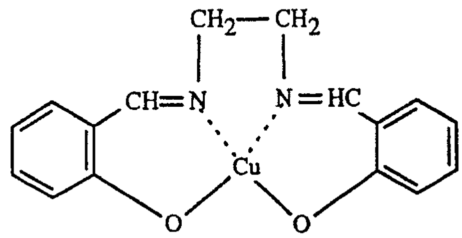

Flotte 4: 0,5 % der Verbindung der Formel (103) und 0,03 % der Verbindung der Formel (109).

In die so präparierten Färbeflotten geht man mit den Trikotmustern bei 40° ein, behandelt etwa 5 Minuten, heizt mit 1,5°/Minute auf 95° auf, behandelt bei dieser Temperatur weitere 20 Minuten, setzt 2 % 80%-ige Essigsäure zu und färbt weitere 30 Minuten. Dann kühlt man auf 60° ab, spült mit kaltem Wasser, zentrifugiert und trocknet bei 80° im Umluftofen.

Die Färbungen der Trikot-Muster werden auf ihre Lichtechtheit nach SN-ISO 105 B02 (=Xenon) und DIN 75.202 (= Fakra) geprüft. Die Ergebnisse sind in Tabelle 2 zusammengefasst.

Tabelle 2:

| Behandlung | Lichtechtheiten | | | Bemerkung |
|---|---|---|---|---|
| | XENON | FAKRA 72 h* | FAKRA 216 h* | |
| Flotte 1 | 6 | 1 H | 1 H | Nach Fakra 216 h belichtetes Muster ist nicht mehr reissfest |
| Flotte 2 | 6-7 | 2+GH | 1-2+GH | — |
| Flotte 3 | 6 | 4 | 3-4 | — |
| Flotte 4 | 6-7 | 4-5 | 4 | — |

*Fakra 72 h = ein Prüfcyclus
*Fakra 216 h = drei Prüfcyclen

Beispiele 5 und 6 : Drei Muster eines Wollgewebes (Sèrge) von je 10 g werden wie in Beispiel 1 beschrieben gefärbt. Es werden jedoch 0,05 % des Farbstoffes der Formel

(110) 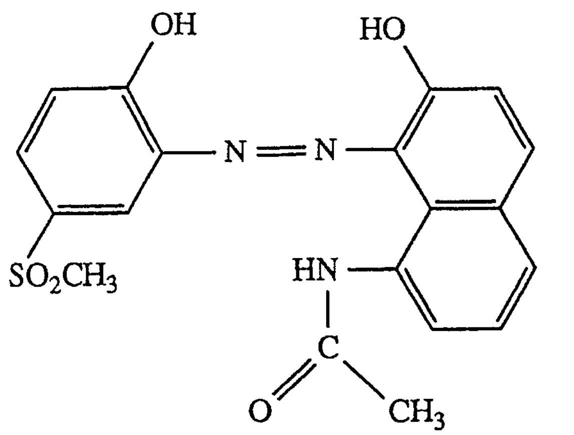

1:2 - Cr-Komplex

0,08 % des Farbstoffes der Formel (III)

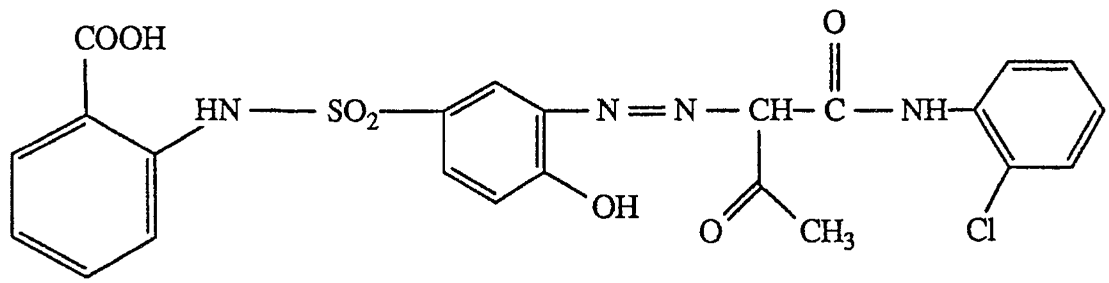

1:2 Cr-Komplex

und 0,005 % des Farbstoffes der Formel

(112)

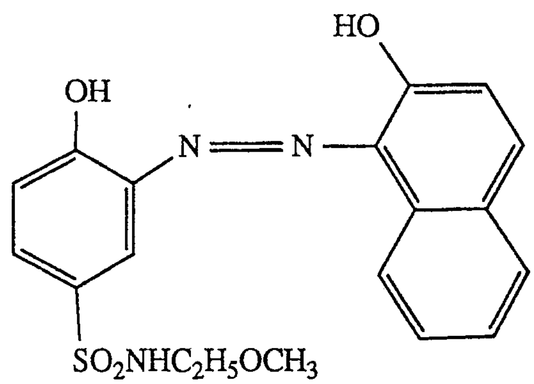

1:2 Co-Komplex

und je 0,75 % der Verbindungen der Formeln (101) und (102) eingesetzt und eine Graufärbung erstellt.

Die Färbungen werden auf Ihre Lichtechtheiten nach SN-ISO 105-B02(=XENON) und DIN 75.202 (FAKRA) geprüft. Weiterhin werden flächige Muster (13 x 4,5 cm) der Graufärbungen 144 Stunden nach DIN 75.202 belichtet und nach SN 198.461 auf Reissfestigkeit und Dehnung geprüft. In Tabelle 3 sind die Ergebnisse aufgeführt:

Tabelle 3

| Färbung | Lichtechtheiten | | | Reissfestigkeit/Dehnung (%)* nach 144 Stunden FAKRA | |
|---|---|---|---|---|---|
| | XENON | FAKRA 72 Stunden | FAKRA 144 Stunden | | |
| 1 | 5 | 2-3G | 1-2G | 38,1 | 27,5 |
| 2 | 5-6 | 3-4 | 2-3G | 45,2 | 35,8 |
| 3 | 5-6 | 4 | -3 | 46,1 | 37,1 |

* nach SN 198.461; auf Ausgangsfärbungen bezogen (=100%)

Es ist ersichtlich, dass mit den Verbindungen der Formeln (101) und (102) sowohl die Färbung als auch die Wolle stabilisiert werden, wodurch deren Lichtechtheiten und textilmechanischen Eigenschaften verbessert werden.

**Ansprüche**

1. Monosulfonierte 2-(2'-Hydroxyphenyl)benzotriazole der Formel

(1) 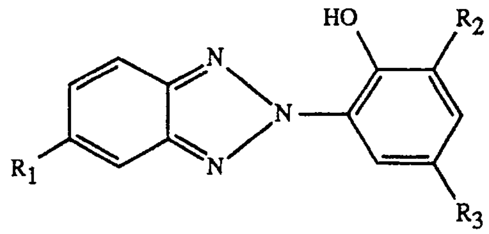 ,

worin $R_1$ Wasserstoff oder Chlor und von $R_2$ und $R_3$ der eine Chlor oder Niederalkyl und der andere einen Rest der Formel

(2) 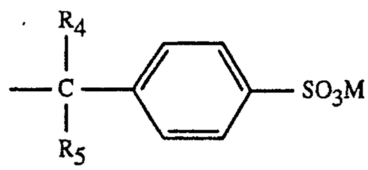

bedeutet, worin $R_4$ und $R_5$ unabhängig voneinander Wasserstoff oder Methyl und M Wasserstoff, Alkalimetall oder Ammonium bedeuten.

2. Monosulfonierte 2-(2'-Hydroxyphenyl)benzotriazole gemäss Anspruch 1, worin $R_2$ Niederalkyl und $R_3$ einen Rest der Formel (2) bedeuten.

3. Monosulfonierte 2-(2'-Hydroxyphenyl)benzotriazole gemäss Anspruch 1, worin $R_2$ einen Rest der Formel (2) und $R_3$ Niederalkyl bedeuten.

4. Verfahren zur Herstellung von monosulfonierten 2-(2'-Hydroxyphenyl)-benzotriazolen gemäss Anspruch 1 durch Sulfonierung einer Verbindung der Formel

(3) 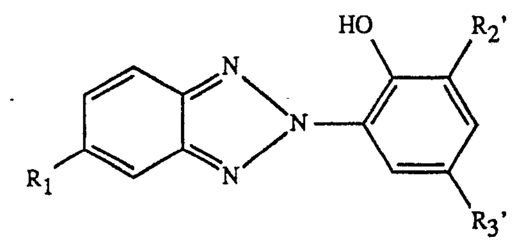 ,

worin $R_1$ die in Formel (1) angegebene Bedeutung hat und von $R_2'$ und $R_3'$ der eine Chlor oder Niederalkyl und der andere einen Rest der Formel

(4) 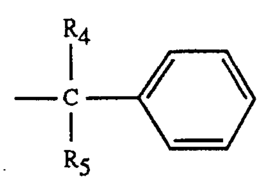

bedeutet, worin $R_4$ und $R_5$ die in Formel (2) angegebene Bedeutung haben.

5. Verfahren gemäss Anspruch 4, dadurch gekennzeichnet, dass die Sulfonierung bei einer Temperatur zwischen 0 und 30° C durchgeführt wird.

6. Verfahren gemäss einem der Ansprüche 4 oder 5, dadurch gekennzeichnet, dass man als Sulfonierungsagens konzentrierte Schwefelsäure, Schwefelsäuremonohydrat oder Oleum verwendet

7. Verwendung von monosulfonierten 2-Hydroxyphenylbenzotriazolen gemäss Anspruch 1 als UV-Absorber für Polyamidfasern, Wolle und deren Mischungen.

Europäisches Patentamt

# EUROPÄISCHER RECHERCHENBERICHT

Nummer der Anmeldung

EP 90 81 0667

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 800 942 (ALLIED CORP.)<br>* Insgesamt *<br>– – – | 1-7 | C 07 D 249/20<br>D 06 M 13/352 |
| A | EP-A-0 006 564 (CIBA-GEIGY)<br>* Insgesamt *<br>– – – – – | 1-7 | |

| RECHERCHIERTE SACHGEBIETE (Int. Cl.5) |
|---|
| C 07 D 249/00 |

Der vorliegende Recherchenbericht wurde für alle Patentansprüche erstellt

| Recherchenort | Abschlußdatum der Recherche | Prüfer |
|---|---|---|
| Den Haag | 05 Dezember 90 | ALLARD M.S. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument